# FASCICULE DE BREVET EUROPEEN

(11) **EP 1 202 763 B1**
(45) Date de publication et mention de la délivrance du brevet: **15.09.2004**
(21) Numéro de dépôt: 00946041.1
(22) Date de dépôt: 30.06.2000
(51) Int. Cl.: A61M 5/30

(54) **SERINGUE SANS AIGUILLE AVEC INJECTEUR A ELEMENTS SUPERPOSES**
NADELLOSE SPRITZE MIT GESTAPELTEN INJEKTORELEMENTEN
NEEDLELESS SYRINGE COMPRISING AN INJECTOR WITH STACKED ELEMENTS

(30) Priorité: 16.07.1999 FR 9909253
(43) Date de publication de la demande: 08.05.2002
(73) Titulaire: CROSSJECT, 75181 Paris Cedex 04 (FR)
(72) Inventeur: ALEXANDRE, Patrick, F-70100 Gray (FR); BROUQUIERES, Bernard, F-83100 Toulon (FR); NAVELIER, Alain, F-83390 Pierrefeu du Var (FR); ROLLER, Denis, F-91590 La Ferte Alais (FR)
(74) Mandataire: Waligorski, Carol
(86) Numéro de dépôt international: PCT/FR2000/001850
(87) Numéro de publication internationale: WO 2001/005453

(56) Documents cités:
- DE-A- 19 607 922
- FR-A- 1 378 829
- US-A- 3 788 315
- US-A- 5 074 843

## Description

La présente invention est dans le domaine des seringues sans aiguille utilisées pour les injections intradermiques, sous-cutanées ou intramusculaires de principe actif liquide à usage thérapeutique en médecine humaine ou vétérinaire.

Dans ce domaine, pour augmenter l'efficacité de l'injection on utilise des seringues avec, à leur partie aval appliquée sur la peau ou très proche de la peau du sujet, un injecteur comportant plusieurs conduits afin de distribuer le liquide à injecter sur plusieurs points répartis sur une surface relativement importante. Cette solution a aussi l'avantage de réduire la douleur et supprimer d'éventuelles altérations superficielles ou sous-cutanées dues à une trop grande quantité de liquide injecté en un seul point.

Pour augmenter l'efficacité de l'injection on joue aussi sur la forme du jet : on contrôle la distance de cohérence du jet et on recherche une solution intermédiaire entre un jet très cohérent, comme pour la découpe par jet qui pénètrerait très profondément et déchirerait dangereusement la peau et un jet qui nébulise le liquide et dont les fines gouttelettes ne pénètrent pas dans la peau.

Le brevet US 3 802 430 décrit une seringue sans aiguille dans laquelle le liquide à injecter est refoulé par un piston repoussé par des gaz produits par un générateur pyrotechnique ; cette seringue comporte cinq conduits parallèles à l'axe de la seringue et de sections transversales circulaires . Le brevet US 3 788 315 décrit une seringue dans laquelle le piston de refoulement du liquide est repoussé par la détente de gaz ou d'un ressort comprimés. Cette seringue comporte six conduits de sections transversales circulaires et dont les axes sont légèrement divergents de l'axe de la seringue. Dans ces exemples, bien que répartissant le liquide sur plusieurs points, les conduits restent assez proches les uns des autres ; de plus la simplicité de la forme de ces conduits montre que ces conduits ne sont pas optimisés pour contrôler la longueur de cohérence du jet qui, elle, est un facteur important pour la performance de l'injecteur dans cette application particulière.

D'une façon plus générale les problèmes que pose la réalisation d'injecteur pour seringue sont des problèmes de résistance mécanique, de performances comme nous venons de l'évoquer et de coût.
En effet l'injecteur, placé à la partie aval de la seringue, ne doit pas se déformer sous l'effet de la pression du liquide lors de l'injection : l'injecteur doit être relativement épais, et cela d'autant plus que les conduits sont répartis sur une grande surface. Le problème va être de réaliser des conduits en général très fins sur de grandes épaisseurs.
La performance de l'injecteur réside dans la possibilité de contrôler la distance de cohérence des jets sortant des conduits ou buses, pour des conditions prédéterminées d'utilisation(nature du liquide, pression d'injection) par des conduits de sections transversales appropriées. Cette section transversale appropriée a pour but de créer un champ de turbulence dans l'écoulement tel que, à faible distance de la sortie de l'injecteur, le jet reste cohérent c'est-à-dire qu'il est assez fin et rapide pour percer et pénétrer dans la peau du sujet à traiter, ensuite le jet perd très rapidement de sa cohérence : il éclate pour diffuser au mieux le principe actif sous la peau. Le problème est de réaliser simplement, non seulement des conduits fins sur de grandes épaisseurs mais surtout des conduits avec des sections transversales appropriées.
Enfin le coût de fabrication devient un facteur très important lorsqu'il s'agit de seringues fabriquées en grande série, notamment pour des seringues jetables.

La présente invention concerne une seringue selon la revendication 2 sans aiguille pour l'injection intradermique, sous-cutanée ou intramusculaire d'un principe actif liquide initialement placé entre, d'une part un injecteur selon la revendication 1 comportant au moins un conduit ou une buse d'injection, ledit injecteur étant placé en contact de la peau ou très proche de la peau du sujet à traiter et d'autre part une paroi déplaçable sous l'effet d'un système propulsif assurant la mise en pression et l'expulsion du principe actif au travers de l'injecteur placé à l'extrémité aval de la seringue, et telle que ledit injecteur comprend un support avec au moins un logement dans lequel sont empilées des plaques comportant chacune un même nombre d'orifïces, les dits orifices des différentes plaques étant alignés pour former au moins une buse à travers l'empilement. Plus précisément, chaque orifice de chaque plaque est aligné avec un orifice correspondant d'une plaque adjacente, la succession des orifices des différentes plaques formant une buse d'injection.
Dans cette invention par principe actif liquide nous entendrons essentiellement un liquide plus ou moins visqueux, ou un mélange de liquides, ou un gel. Le principe actif pourra être un solide mis en solution dans un solvant approprié pour l'injection. Le principe actif pourra être un solide sous forme pulvérulente mis en suspension, plus ou moins concentrée, dans un liquide approprié. La granulométrie du principe actif solide doit être adaptée ainsi que la forme du conduit pour éviter les bouchages des conduits.
Les plaques ont des formes géométriques simples, par exemple des formes polygonales, elliptiques ou circulaires. Les logements du support dans lesquels sont empilées lesdites plaques ont bien sûr des formes conjuguées qui permettent l'emboîtement des plaques. Ces plaques sont en général planes, à faces parallèles pour plus de simplicité ; mais pour certaines plaques au moins une face peut être convexe ou concave. Enfin ces plaques sont d'épaisseur égalé ou non.
Les orifices dans une plaque sont des trous à travers la plaque. Ces trous sont soit à symétrie de révolution : section transversale circulaire, soit ne présentent pas cette symétrie : section transversale polygonale (triangle, carré, ···) ou mixte (c'est-à-dire une section transversale avec certains côtés non rectilignes). On assimilera à des trous des rainures faites sur le bord d'une plaque. Les différentes plaques sont empilées dans un logement de façon que les trous ou rainures de chaque plaque se suivent pour réaliser des buses à travers l'empilement.

Dans une réalisation particulière la seringue sans aiguille est telle que ledit injecteur comprend un support avec un seul logement dans lequel_sont empilées des plaques comportant chacune un même nombre d'orifices, lesdits orifices étant alignés pour former au moins une buse à travers l'empilement.

Préférentiellement les orifices en correspondance dans les plaques ont des formes géométriques différentes de façon à réaliser, par leur succession dans l'empilement, des buses de section transversale évolutive. Cette section transversale évolutive est obtenue en combinant dans l'empilement des orifices de formes de sections différentes ; par exemple des orifices cylindriques, tronconiques, ou avec des profils courbes, ou une succession de conduits et de cavités pour réaliser une buse à section transversale évolutive.

Avantageusement, les plaques comportent des moyens de calage angulaire desdites plaques dans le logement correspondant, de façon que les orifices se correspondent et se succèdent pour réaliser une buse à section transversale évolutive. Le calage angulaire se fait par des formes conjuguées du logement et des plaques si elles ont des formes polygonales. Si les logements ont des formes de révolution, le calage angulaire sera assuré par un pion traversant toutes les plaques et disposé latéralement ou par une nervure du logement s'engageant dans une encoche des plaques ou par toute autre dispositif équivalent.

De préférence les formes des logements et des plaques sont telles que, lors de l'injection, la pression du liquide bloque les plaques contre un épaulement du logement, ou les bloque par une conicité appropriée : partie de section plus petite vers l'aval.

Dans une première réalisation de la seringue, au moins la plaque la plus en aval est emmanchée à force dans un logement pour assurer l'étanchéité.
Dans une deuxième réalisation de la seringue, au moins la plaque la plus en aval est collée dans son logement.

Avantageusement le support des plaques est l'extrémité aval de la seringue elle-même. Cette extrémité aval est aménagée, épaulement ou conicité, pour recevoir l'empilement des plaques.

Dans une variante de la seringue selon l'invention, ledit injecteur comprend au moins une plaque constituant une membrane traversable intercalée entre deux plaques consécutives.
En l'absence de pression cette membrane traversable obstrue la buse formée par les orifices alignés des plaques empilées : la membrane empêche ainsi des pertes de liquide du fait de secousses ou de manipulations brutales de la seringue. Au moment de l'injection, le liquide est mis en pression par la paroi déplaçable activée par le système propulsif, le liquide va traverser ladite membrane.
Dans une première réalisation la membrane est assez mince pour qu'elle se perce, en regard des conduits ou buses d'injection, lorsque le liquide est mis sous forte pression pour faire l'injection.
Dans une deuxième réalisation la membrane comporte une zone de moindre épaisseur, en regard de chaque conduit d'injection ; chacune de ces zones se perce, comme précédemment, au moment de la mise en pression. Pour le bon fonctionnement de cette réalisation la membrane doit être bien positionnée, par des dispositifs appropriés, pour que chaque zone de moindre épaisseur soit en vis à vis d'un conduit d'injection. Une zone de moindre épaisseur est une cavité borgne faite dans la membrane. Une des formes les plus simples est celle d'un cône ou d'un tronc de cône.
Dans une troisième réalisation la membrane comporte un préperçage en regard de chaque conduit d'injection. L'élasticité de l'élastomère de la membrane maintient fermé chacun des préperçages et éventuellement cette fermeture est étanche. Lorsque le liquide est mis en pression par le déclenchement du système propulsif, chaque préperçage s'ouvre.

La présente invention concerne aussi un injecteur, pour seringue sans aiguille. Ledit injecteur comprend un support avec au moins un logement dans lequel sont empilées des plaques comportant chacune un même nombre d'orifices, lesdits orifices étant alignés pour former au moins une buse à travers l'empilement.

Enfin la présente invention concerne un procédé de fabrication d'un injecteur, pour une seringue sans aiguille. Ce procédé comporte les étapes suivantes :
- fabrication d'un support comportant au moins un logement,
- fabrication de plaques comportant toutes le même nombre d'orifices, de géométries convenablement choisies,
- empilement et calage des plaques selon un ordre prédéterminé dans chaque logement pour former au moins une buse à travers l'empilement.

Une seringue selon l'invention résout les problèmes posés. Pour la résistance de l'injecteur l'augmentation de l'épaisseur ne présente pas de difficulté vis à vis de la réalisation de conduits fins, avec des sections transversales évolutives ou non, sur de grandes épaisseurs.
L'invention permet de faire de façon simple, pour s'adapter aux conditions prédéterminés d'utilisation, le contrôle de la distance de cohérence des jets sortant des buses.
Pour l'aspect coût, l'injecteur comporte des éléments de formes simples et faciles à réaliser, l'assemblage de ces éléments est, lui aussi, simple et surtout se prête à une automatisation poussée.
La seringue selon l'invention présente de plus un avantage indéniable du point de vue de la sécurité en cas d'une utilisation anormale. Par exemple, si la seringue est dirigée vers un visage et déclenchée accidentellement, les jets n'auront pas d'autres effets que d'arroser ledit visage de principe actif, sans aucun effet mécanique de percement si la seringue n'est pas en contact (ou très proche) du visage. Cet avantage est lié à la maîtrise de la distance de cohérence du jet.

La présente invention va être décrite plus en détail à l'aide des figures suivantes.
La figure **1** représente, en coupe longitudinale partielle une seringue selon l'invention ; l'injecteur de cette seringue comprend plusieurs logements avec des empilements identiques de plaques avec chacune un orifice central.
La figure **2** représente, en coupe longitudinale, le détail d'un logement avec l'empilement de plaques de l'exemple précédent.
La figure **3** représente, en coupe longitudinale partielle l'extrémité aval d'une seringue dans un autre exemple de réalisation.
La figure **4** représente, en coupe longitudinale, le détail d'un logement avec l'empilement de plaques dont une constitue une membrane traversable.

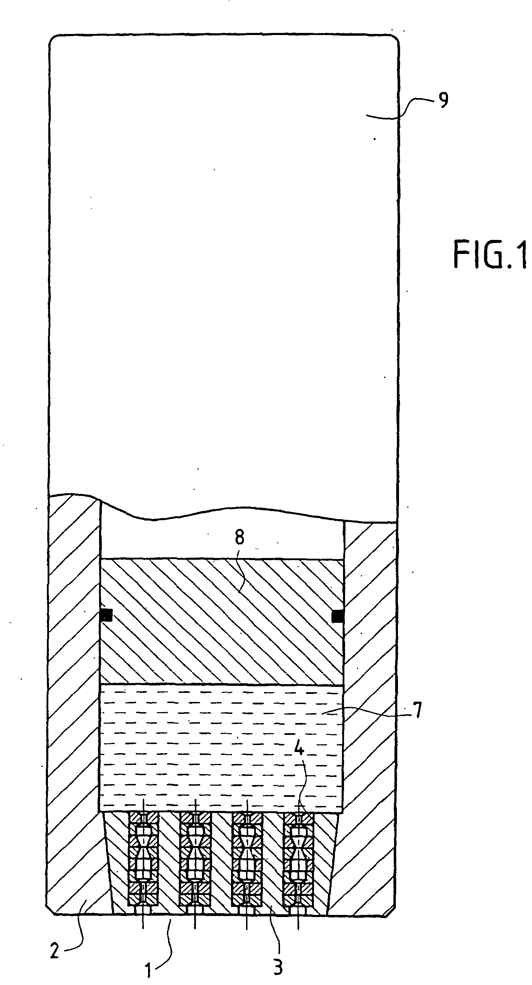

La figure 1 représente schématiquement une seringue sans aiguille pour l'injection de principe actif liquide. Une telle seringue est en général cylindrique et comporte un réservoir contenant le principe actif 7. Ce réservoir est fermé à une extrémité, que nous avons appelée extrémité aval 2 par un injecteur 1 comportant au moins une buse d'injection. Cet injecteur est en général en appui sur la peau du sujet à traiter, ou maintenue à très faible distance, la peau n'est pas représentée sur ce dessin. Cet injecteur est l'extrémité du réservoir ou est une pièce rapportée 3 fixée sur cette extrémité du réservoir, par des moyens appropriés. L'autre extrémité du réservoir est fermée par une paroi déplaçable, par exemple un piston 8 comportant des moyens pour assurer l'étanchéité tels qu'un joint torique. Enfin la seringue comporte un système propulsif 9 avec un dispositif de déclenchement pour déplacer le piston et injecter le liquide. Parmi les systèmes propulsifs utilisables et sans entrer dans leurs détails nous citerons un générateur pyrotechnique de gaz, comme décrit dans le brevet US 3 802 430 précédemment cité, nous citerons aussi la détente de gaz ou d'un ressort comprimés, comme décrit dans le brevet US 3 788 315. Il est évident que les seringues selon l'invention peuvent être équipées d'un de ces types de système propulsif pour déplacer le piston.

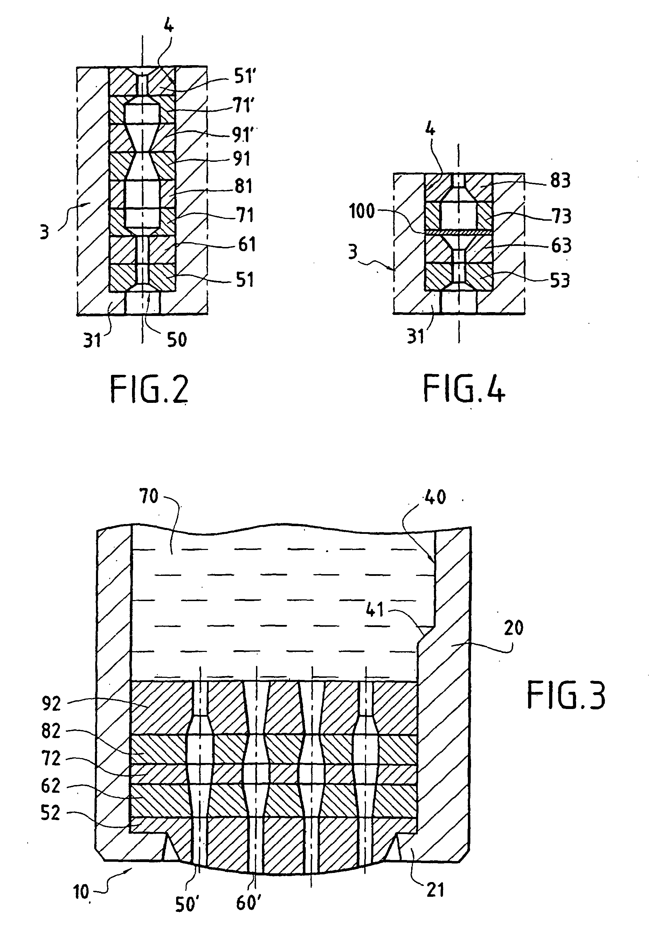

Le support 3 de l'injecteur 1 représenté sur la figure 1 est une pièce tronconique emmanchée dans l'extrémité 2 de la seringue. Le support 3 comporte dans cet exemple huit logements identiques répartis sur deux cercles concentriques, ces logements 4 sont cylindriques, ouverts à leurs deux extrémités, l'ouverture de l'extrémité aval étant de diamètre plus petit pour faire un épaulement 31. Dans chaque logement 4 on trouve (voir figure 2) un empilement identique de huit plaques, (51,61,71,81,91,51',71',91') toutes de même diamètre extérieur et de même épaisseur. Chaque plaque comporte un orifice central de forme simple : un cylindre de petit diamètre (plaque 61), un cylindre de grand diamètre (plaque 81), un tronc de cône (plaque 91 et 91' qui différent par l'orientation de là conicité vis à vis de l'écoulement de liquide ) ; un petit tronc de cône se raccordant à un cylindre (plaques 51 et 51'), un tronc de cône se raccordant à un cylindre (plaques 71 et 71'). L'empilement de ces plaques selon un ordre particulier dans un logement 4 réalise une buse 50 de section transversale évolutive, la succession des différentes cavités permettant de contrôler la distance de cohérence du jet de liquide sortant de ce conduit.

Dans cet exemple les plaques ont environ 3mm de diamètre extérieur et environ 1mm d'épaisseur. Les orifices ont un diamètre compris entre environ 0,1mm et environ 0,6mm. Le support 3 a un diamètre de environ 20mm à environ 30mm.

La figure 3 représente, en coupe longitudinale, l'extrémité aval d'une autre réalisation d'une seringue selon l'invention. Dans cette réalisation le support de l'injecteur 10 est un épaulement 21 de l'extrémité aval 20 de la seringue elle-même. Dans le logement unique 40, ici cylindrique, de l'extrémité 20 de la seringue sont empilées des plaques 52,62,72,82 et 92 retenues par l'épaulement 21 de l'extrémité 20. Les plaques 62,72,82 et 92 sont planes, de même diamètre extérieur mais d'épaisseurs différentes. La plaque 52, la plus en aval, a une forme un peu plus complexe, elle a une partie aval qui se centre dans l'ouverture de l'épaulement 21 avec une face aval légèrement convexe ; sa partie opposée a le même diamètre extérieur que celui des autres plaques et elle est plane.

Toutes les plaques comportent le même nombre d'orifices. Ces orifices sont de formes simples dans cet exemple, des cylindres, dès troncs de cône. Ces formes se combinent pour réaliser des buses à sections évolutives. Dans cet exemple deux types de buses 50,60 sont représentés. Un dispositif permet d'orienter convenablement les plaques les unes par rapport aux autres pour réaliser les conduits. Ce dispositif est une nervure 41 du logement 40 s'engageant dans une encoche latérale des différentes plaques. L'empilement des plaques 52,62,72,82,92 est emmanché forcé dans le logement 40 de l'extrémité 20 de la seringue.

Les matériaux pour réaliser la seringue et les différentes parties de l'injècteur seront choisis parmi les matériaux compatibles et agrées pour un usage médical ; sans prétendre être exhaustif nous citerons par exemple des matériaux plastiques tels que les polycarbonates, des polytétrafluoréthylènes ; des métaux : tels que des inox, ou du verre à usage médical (par exemple du type I ou du type II).

Sur la figure 4 on retrouve un logement 4 semblable à celui décrit dans la figure 2. Dans ce logement sont empilées des plaques 53,63,73,83 perforées semblables à celles de l'empilement de la figure 2 et une plaque constituant une membrane traversable 100. Dans cet exemple la membrane 100 est réalisée par une feuille mince d'un matériau qui va se déchirer lors de la mise en pression du liquide au moment de l'injection. Cette membrane peut aussi comporter une zone de moindre épaisseur qui va faciliter le perçage de la membrane en cet endroit, lors de la mise en pression du liquide pour l'injection. Selon une autre variante la membrane comporte des préperçages, alignés avec les orifices des plaques 53,63,73,83, l'élasticité du matériau de la membrane maintient les préperçages fermés en l'absence de pression. La pressurisation du liquide, pour l'injection, écarte les bords des préperçages pour laisser passer le liquide.
La membrane traversable est fabriquée avec un élastomère ou un polymère compatible avec le principe actif liquide ; son épaisseur est comprise entre environ 0,2mm et environ 1,5mm.

## Revendications

1. Injecteur (1,10) pour seringue sans aiguille, ledit injecteur comprenant un support (3,20) **caractérisé en ce que** ledit support (3,20) comprend au moins un logement (4,40) dans lequel sont empilées des plaques (51,61...,91,51',71',91',52,...,92) comportant chacune un même nombre d'orifices, lesdits orifices étant alignés pour former au moins une buse (50,50',60') à travers l'empilement.

2. Seringue sans aiguille pour l'injection d'un principe actif (7,70), ladite seringue comprenant à son extrémité aval (2,20) un injecteur (1,10) selon la revendication 1, le principe actif (7, 70) et une paroi déplaçable (8) sous l'effet d'un système propulsif (9) assurant la mise en pression et l'expulsion du principe actif au travers dudit injecteur.

3. Seringue sans aiguille selon la revendication 2 **caractérisée en ce que** ledit support comprend un seul logement (40) dans lequel sont empilées des plaques (52,62,...,92) comportant chacune un même nombre d'orifices, lesdits orifices étant alignés pour former au moins une buse (50',60') à travers l'empilement.

4. Seringue sans aiguille selon la revendication 2 ou 3 **caractérisée en ce que** les orifices dans les plaques (51,61,...,91,51',71',91',52,...,92) ont des formes géométriques simples de façon à réaliser des buses (50,50',60') de section transversale évolutive.

5. Seringue sans aiguille selon la revendication 4 **caractérisée en ce que** les logements (4,40) et les plaques (51,61,...,91,51',71',91',52,...,92 comportent des moyens de calage angulaire desdites plaques dans le logement correspondant.

6. Seringue sans aiguille selon la revendication 5 **caractérisée en ce que** au moins la plaque la plus en aval (51,52) est emmanchée à force dans son logement (4,40).

7. Seringue sans aiguille selon la revendication 5 **caractérisée en ce que** au moins la plaque la plus aval (51,52) est collée dans son logement (4,40).

8. Seringue sans aiguille selon l'une des revendications 2 à 7 **caractérisée en ce que** le support des plaques est l'épaulement (21) de l'extrémité aval (20) de la seringue.

9. Seringue selon l'une des revendications 2 à 8 **caractérisée en ce que** ledit injecteur comprend au moins une plaque constituant une membrane traversable (100) intercalée entre deux plaques consécutives (63,73).

10. Procédé de fabrication d'un injecteur (1,10), pour une seringue sans aiguille, comportant les étapes suivantes :
- fabrication d'un support (3,20) comportant au moins un logement (4,40),
- fabrications de plaques (51,61,...,91,51',71',91', 52,...92) comportant toutes le même nombre d'orifice,
- empilement et calage des plaques (51,61,...91,51', 71',91',52,...,92)
selon un ordre prédéterminé dans chaque logement (4,40) pour former au moins une buse (50,50',60') à travers l'empilement.

## Patentansprüche

1. Injektor (1, 10) für eine nadellose Spritze, wobei der Injektor einen Träger (3, 20) aufweist, **dadurch gekennzeichnet, dass** der Träger (3, 20) mindestens einen Sitz (4, 40) aufweist, in dem Platten (51, 61, ..., 91, 51', 71', 91', 52, ..., 92) gestapelt sind, je die gleiche Anzahl von Öffnungen aufweisen, wobei die Öffnungen ausgerichtet sind, um durch den Stapel hindurch mindestens eine Düse (50, 50', 60') zu bilden.

2. Nadellose Spritze zum Spritzen eines Wirkstoffs (7, 70), wobei die Spritze an ihrem stromabwärts liegenden Ende (2, 20) einen Injektor (1, 10) nach Anspruch 1, den aktiven Wirkstoff (7, 70) und eine unter der Wirkung eines Antriebssystems (9) verschiebbare Wand (8) aufweist, die das Unterdrucksetzen und den Ausstoß des Wirkstoffs durch den Injektor hindurch gewährleistet.

3. Nadellose Spritze nach Anspruch 2, **dadurch gekennzeichnet, dass** der Träger einen einzigen Sitz (40) aufweist, in dem Platten (52, 62, ..., 92) gestapelt sind, die alle die gleiche Anzahl von Öffnungen aufweisen, wobei die Öffnungen fluchtend ausgerichtet sind, um durch den Stapel hindurch mindestens eine Düse (50', 60') zu bilden.

4. Nadellose Spritze nach Anspruch 2 oder 3, **dadurch gekennzeichnet, dass** die Öffnungen der Platten (51, 61, ..., 91, 51', 71', 91', 52, ..., 92) einfache geometrische Formen haben, um Düsen (50, 50', 60') mit fortschreitendem Querschnitt herzustellen.

5. Nadellose Spritze nach Anspruch 4, **dadurch gekennzeichnet, dass** die Sitze (4, 40) und die Platten (51, 61,... ,91, 51', 71', 91', 52, ..., 92) Mittel zum winkelmäßigen Verkeilen der Platten im entsprechenden Sitz aufweisen.

6. Nadellose Spritze nach Anspruch 5, **dadurch gekennzeichnet, dass** zumindest die am weitesten stromabwärts liegende Platte (51, 52) in ihren Sitz (4, 40) presseingepasst wird.

7. Nadellose Spritze nach Anspruch 5, **dadurch gekennzeichnet, dass** zumindest die am weitesten stromabwärts liegende Platte (51, 52) in ihren Sitz (4, 40) geklebt wird.

8. Nadellose Spritze nach einem der Ansprüche 2 bis 7, **dadurch gekennzeichnet, dass** der Träger der Platten die Schulter (21) des stromabwärts liegenden Endes (20) der Spritze ist.

9. Nadellose Spritze nach einem der Ansprüche 2 bis 8, **dadurch gekennzeichnet, dass** der Injektor mindestens eine Platte aufweist, die eine durchquerbare Membran (100) bildet, die zwischen zwei aufeinanderfolgende Platten (63, 73) eingefügt ist.

10. Verfahren zur Herstellung eines Injektors (1, 10) für eine nadellose Spritze, das die folgenden Schritte aufweist:
- Herstellung eines Trägers (3, 20) mit mindestens einem Sitz (4, 40),
- Herstellung von Platten (51, 61, ..., 91, 51', 71', 91', 52, ..., 92), die alle die gleiche Anzahl von Öffnungen aufweisen,
- Stapeln und Verkeilen der Platten (51, 61...91, 51, 71', 91', 52, ..., 92) in einer vorgegebenen Ordnung in jedem Sitz (4, 40), um durch den Stapel hindurch mindestens eine Düse (50, 50', 60') zu bilden.

## Claims

1. An injector (1, 10) for a needleless syringe, said injector comprising a support (3, 20), **characterised in that** said support (3, 20) comprises at least one receptacle (4, 40) in which are stacked plates (51, 61, ..., 91, 51', 71', 91', 52, ..., 92) each comprising the same number of orifices, said orifices being aligned to form at least one nozzle (50, 50', 60') through the stack.

2. A needleless syringe for the injection of an active substance (7, 70), said syringe comprising at its downstream end (2, 20) an injector (1, 10) according to claim 1, the active substance (7, 70) and a wall (8) which is displaceable under the action of a propulsive system (9) ensuring pressurisation and expulsion of the active substance through said injector.

3. A needleless syringe according to claim 2, **characterised in that** said support comprises a single receptacle (40) in which are stacked plates (52, 62, ..., 92) each comprising the same number of orifices, said orifices being aligned to form at least one nozzle (50', 60') through the stack.

4. A needleless syringe according to claim 2 or claim 3, **characterised in that** the orifices in the plates (51, 61, ..., 91, 51', 71', 91', 52, ..., 92) have simple geometric shapes such as to produce nozzles (50, 50', 60') of changing cross-section.

5. A needleless syringe according to claim 4, **characterised in that** the receptacles (4, 40) and the plates (51, 61, ..., 91, 51', 71', 91', 52, ..., 92) comprise means for angular wedging of said plates in the corresponding receptacle.

6. A needleless syringe according to claim 5, **characterised in that** at least the plate furthest downstream (51, 52) is pressed by force into its receptacle (4, 40).

7. A needleless syringe according to claim 5, **characterised in that** at least the plate furthest downstream (51, 52) is glued into its receptacle (4, 40).

8. A needleless syringe according to one of claims 2 to 7, **characterised in that** the plates are supported by the shoulder (21) of the downstream end (20) of the syringe.

9. A syringe according to one of claims 2 to 8, **characterised in that** said injector comprises at least one plate constituting a permeable membrane (100) interposed between two consecutive plates (63, 73).

10. A process for manufacturing an injector (1, 10), for a needleless syringe, comprising the following stages:
- manufacture of a support (3, 20) comprising at least one receptacle (4, 40),
- manufacture of plates (51, 61, ..., 91, 51', 71', 91', 52, ..., 92) all comprising the same number of orifices,
- stacking and wedging of the plates (51, 61, ..., 91, 51', 71', 91 ', 52, ..., 92)
in a predetermined order in each receptacle (4, 40) to form at least one nozzle (50, 50', 60') through the stack.
